# EUROPÄISCHE PATENTANMELDUNG

(11) **EP 1 444 950 A1**
(43) Veröffentlichungstag der Anmeldung: **11.08.2004**
(21) Anmeldenummer: 03388009.7
(22) Anmeldetag: 07.02.2003
(51) Int. Cl.: A61B 5/12, A61B 5/0484, G06F 17/00

(54) **Verfahren zum objektiven Nachweis von Auditory Steady-State Responses (ASSR) im Zeitbereich**

(71) Anmelder: Ekkehard Stürzebecher, 15370 Petershagen (DE)
(72) Erfinder: Ekkehard Stürzebecher, 15370 Petershagen (DE)
(74) Vertreter: Christensen, Mikael T.

(57) **Zusammenfassung**

Die Erfindung betrifft ein Verfahren zum objektiven Nachweis von Auditory Steady-State Responses (ASSR) im Zeitbereich, wobei die einzelnen Epochen in den Frequenzbereich transformiert werden. Hierbei kann im Frequenzbereich die Antwort vom Rauschen des Spontan-EEG befreit werden, indem die Amplituden der Spektrallinien, die nur Rauschen enthalten, auf Null gesetzt werden und die Spektrallinien der Harmonischen unbeeinflußt bleiben. Das veränderte Spektrum wird dann in den Zeitbereich rücktransformiert.

## Beschreibung

Die Erfindung betrifft das Gebiet der objektiven Messung des Hörvermögens unter Nutzung von auditorisch evozierten steady-state responses (auditory steady-state responses, ASSR). Das vorgeschlagene Verfahren zum objektiven Nachweis von ASSR im Zeitbereich kann sowohl bei einem ASSR-basierten Neugeborenen-Hörscreening als auch bei der objektiven Hörschwellenmessung auf ASSR-Basis eingesetzt werden.

Es sind zwei verschiedene Arten von ASSR bekannt:
1. click-evozierte ASSR
2. ASSR, die durch einen amplituden- oder frequenzmodulierten Dauerton evoziert werden, die auch als amplitude-modulation following response (AMFR) bezeichnet werden.
   Beide Arten von ASSR werden im Frequenzbereich durch sog. Harmonische (eine Grundwelle und mehrere Oberwellen) beschrieben. Die Frequenz der Grundwelle entspricht der Click-Reizrate bzw. der Frequenz des Modulationssignals. Die Frequenzen der Oberwellen ergeben sich als Vielfache der Frequenz der Grundwelle. Das bedeutet, daß die gesamte Antwort durch wenige Spektrallinien repräsentiert wird. Der wesentliche Anteil der durch das Spontan-Elektroenzephalogramm (Spontan-EEG) bedingten Rauschleistung ist dagegen auf die zwischen den Harmonischen liegenden Spektrallinien konzentriert.

Der objektive Nachweis der bekannten ASSR erfolgt bisher ausschließlich im Frequenzbereich. Dafür sind mehrere statistische Verfahren bekannt. (Stapells DR, Makeig S, Galambos R. Auditory steady-state responses: Threshold prediction using phase coherence. Electroencephalography and Clinical Neurophysiology 1987;67:260-270; Valdes JL, Perez-Abalo MC, Martin V, Savio G, Sierra C, Rodriguez E, Lins O. Comparison of statistical indicators for the automatic detection of 80 Hz auditory steady state response (AMFR). Ear and Hearing 1997;18:420-429), die als sog. One-Sample Tests nur die Phase oder auch Phase und Amplitude einer einzelnen Spektrallinie, vorzugsweise der 1. Harmonischen (Grundwelle) auswerten. Hierzu wird das registrierte Zeitsignal in den Frequenzbereich transformiert. Die Länge der transformierten Epochen muß so gewählt werden, daß die Epoche ein ganzzahliges Vielfaches der Periodendauer der Click-Reizrate bzw. der Modulationsfrequenz ist. In dem nach der Transformation vorliegenden Frequenzspektrum wird die der Click-Reizrate bzw. der Modulationsfrequenz entsprechende Spektrallinie (Grundfrequenz) aufgesucht und getestet. Der Vorteil des Antwort-Nachweises im Frequenzbereich gegenüber dem direkten Nachweis im Zeitbereich besteht darin, daß im Spektralbereich der Anteil der Rauschleistung, der durch die zwischen den Harmonischen liegenden Spektrallinien repräsentiert wird, die Response Detection nicht stört, da diese Spektrallinien nicht in die Testung einbezogen werden. Der Nachteil der in den oben genannten Publikationen beschriebenen Methode besteht in der Beschränkung der statistischen Testung auf die Grundfrequenz. ASSR werden jedoch in der Regel nicht allein durch die der Click-Folgefrequenz bzw. der Modulationsfrequenz entsprechende Grundfrequenz, sondern auch durch eine oder mehrere Oberwellen repräsentiert, auf die ein nicht zu vernachlässigender Anteil der Response-Signalleistung entfällt. Ein objektives Nachweisverfahren, das sich nur auf die Grundwelle beschränkt, ist deshalb nicht optimal.
Von Stürzebecher sowie von Stürzebecher et al. werden ebenfalls im Frequenzbereich arbeitende Verfahren beschrieben (Stürzebecher E: "Method for hearing screening of newborn by means of steady-state response evoked with high click rate", European patent application EP 01610060.4.; Stürzebecher, E, Cebulla M, Baag M, Thie R: "Verfahren zur objektiven frequenzspezifischen Hörschwellenbestimmung mittels der Amplitude-Modulation Following Response (AMFR)", European patent application EP1099408 A2 ), die für den statistischen Nachweis der ASSR sowohl die Grundwelle als auch die relevanten Oberwellen verwenden. Die Verfahren nutzen entweder nur die spektralen Phasenwinkel der Harmonischen für den Antwort-Nachweis oder die spektralen Phasenwinkel und die spektralen Amplituden der Harmonischen. Durch die Einbeziehung der höheren Harmonischen wird eine größere Empfindlichkeit für den Antwortnachweis im Vergleich zu den Verfahren, die nur mit der Grundwelle (1. Harmonische) arbeiten, erreicht.

Bei einem im Zeitbereich arbeitenden statistischen Verfahren ist dagegen eine Trennung von Signal und Rauschen, wie sie oben für den Frequenzbereich beschrieben wird, nicht ohne weiteres möglich. Die gesamte Rauschleistung des Spontan-EEG ist der Antwort-Zeitfunktion überlagert und verursacht eine geringere Empfindlichkeit der Response Detection. Diese geringere Empfindlichkeit kann auch durch die bekannten im Zeitbereich arbeitenden statistischen Test mit hoher Test-Power nicht kompensiert werden.

Aufgabe der Erfindung ist es, ein Verfahren zu entwickeln, das es ermöglicht, die im Frequenzbereich mögliche Separierung von Antwort und dem im wesentlichen durch das Spontan-EEG verursachten Rauschen auch im Zeitbereich zu realisieren um so die hohe Test-Power von bekannten statistischen Testverfahren im Zeitbereich zur Geltung zu bringen.

Eine erfindungsgemäße Lösung dieser Aufgabe ist in Patentanspruch 1 angegeben. Weiterbildungen der Erfindung sind in den Unteransprüchen gekennzeichnet.

Die Konzeption der Erfindung besteht darin, daß, wie bei der Detection der Response im Frequenzbereich, die einzelnen Epochen in den Frequenzbereich transformiert werden. Im Frequenzbereich wird die Antwort vom Rauschen des Spontan-EEG befreit. Dafür bestehen zwei Möglichkeiten:
1. Die Amplituden der Spektrallinien, die nur Rauschen enthalten, werden auf Null gesetzt. Die Spektrallinien der Harmonischen bleiben unbeeinflußt. Das so veränderte Spektrum wird in den Zeitbereich rücktransformiert.
2. Alle Spektrallinien, die nur Rauschen enthalten, werden weggelassen. Da heißt, das Spektrum besteht nur noch aus den Harmonischen der Antwort. Das so veränderte Spektrum wird in den Zeitbereich rücktransformiert.

Auf die nach Rücktransformation in den Zeitbereich erhaltene Zeitfunktion der Antwort wird ein im Zeitbereich arbeitender statistischer Test angewendet.

Die Anwendung der erfindungsgemäßen Lösung hat folgende Vorteile:

### 1. Vorteil

Die nach Rücktransformation aus dem Frequenzbereich erhaltene Zeitfunktion enthält im wesentlichen nur noch die Anwort. Der Antwort sind nur geringe Rauschanteile überlagert, die in den Harmonischen enthalten sind. Beide beschriebenen Verfahren zur "Rauschfilterung" sind etwa gleichwertig, das Verfahren 2 hat aber den leichten Vorteil, daß die Rücktransformation geringfügig weniger Rechenaufwand erfordert.
Im Vergleich zur Anwendung des statistischen Tests auf die originale (nicht vom Rauschen befreite) Antwort im Zeitbereich führt die Anwendung eines statistischen Tests auf die rauschbefreite Antwort im Zeitbereich zu einer höheren Nachweisempfindlichkeit. D.h., bei einem Hörscreening mit vorgegebenen Reizpegel wird die Antwort schneller detektiert, dadurch ist der Zeitaufwand für das Screening geringer. Bei einer objektiven Schwellenbestimmung ist eine genauere objektive Schwellenbestimmung möglich, da der Antwortnachweis näher an der Hörschwelle des Patienten gelingt.

### 2.Vorteil

Ein weiterer wichtiger Vorteil der beschriebenen Vorbehandlung der Daten im Frequenzbereich besteht darin, daß zwei Antwort-Zeitfunktionen, die bei simultaner auditorischer Reizung beider Ohren generiert werden, aber im Zeitbereich einander überlagert und deshalb nicht auswertbar sind, nach Vorbehandlung im Frequenzbereich einzeln im Zeitbereich darstellbar und einzeln statistisch testbar sind. Das wird ermöglicht durch unterschiedliche Click-Reizraten für das rechte und linke Ohr.

Weitere Einzelheiten, Merkmale und Vorteile der Erfindung ergeben sich aus der nachfolgenden Beschreibung eines Ausführungsbeispiels

### Ausführungsbeispiel

Bei einem Neugeborenen soll mittels der ASSR auf der Säuglingsstation geprüft werden, ob das Hörvermögen beider Ohren normal ist. Die Untersuchung erfolgt während des natürlichen Schlafs nach der Nahrungsaufnahme.

Die Click-Reizrate für die Prüfung des rechten Ohres beträgt 160 Clicks/s, für die Prüfung des linken Ohres 140 Clicks/s. Für jede der beiden Reizfolgefrequenzen wird vor dem Start der Untersuchung jeweils eine Click-Reizfolge mit einer Länge von 1 Sekunde berechnet und in einem Pufferspeicher abgelegt. Die beiden Click-Folgen für die akustische Stimulation werden durch zyklische Abfrage des Pufferspeichers erzeugt und nach D/A-Konvertierung mit einem Pegel von 40 dBnHL über je einen Hörer auf das rechte und das linke Ohr gegeben. Die Taktfrequenz für die D/A-Konvertierung beträgt 16384/s. Während der akustischen Reizung wird das EEG mittels Klebeelektroden von der Kopfhaut des Säuglings abgeleitet. Die Elektrodenlage ist Vertex/Nacken, Erde: Stirn. Das EEG wird verstärkt und A/D-konvertiert. Der Takt für D/A- und A/D-Konverter muß synchronisiert sein. Im vorliegenden Beispiel beträgt die Taktfrequenz des A/D-Konverters 4096 Hz. Der A/D- Takt wird durch Teilung (Faktor 4) aus dem Takt des D/A-Konverters gewonnen.

Das digitalisierte EEG wird im Signalprozessor des Screeninggerätes fortlaufend in Abschnitte (Epochen) mit einer Länge von 1 s geteilt. Eine bekannte Einrichtung zur Artefaktunterdrückung bewirkt, daß Epochen mit Artefakten nicht in die weitere Auswertung einbezogen werden. Die artefaktfreien Epochen werden mittels FFT in den Frequenzbereich transformiert. Phasenwinkel und spektrale Amplituden der den beiden Grundfrequenzen und den zugehörigen Oberwellen entsprechenden Spektrallinien werden in zwei Datenmatrizen gespeichert. Für jede der beiden Click-Reizraten werden je 5 Harmonische gespeichert. Alle Spektrallinien, die nur EEG-Rauschen enthalten, werden verworfen. Um im Frequenzspektrum Grund- und Oberwellen ohne Seitenbänder zu erhalten, darf jede Epoche nur ganzzahlige Vielfache der Perioden der über das rechte und linke Ohr evozierten SSP enthalten. Das ist durch die Wahl der Epochenlänge und der beiden Click-Folgefrequenzen gewährleistet. Eine FFT der Epochen ist möglich, da die Anzahl der Abtastwerte pro Epoche ein ganzzahliges Vielfaches von 2 ist.

Die auf die 5 Harmonischen reduzierten Spektren der für das rechte und linke Ohr erhaltenen Antworten werden mittels inverser direkter Fouriertransformation in den Zeitbereich rücktransformiert. Danach liegt für jede Epoche eine Zeitfunktion für das rechte Ohr und eine Zeitfunktion für das linke Ohr vor, die in zwei Datenmatrizen gespeichert werden. Die statistische Testung im Zeitbereich im erfolgt mit dem Friedman-Test (Sachs L. Angewandte Statistik. Springer Verlag Berlin, New York, London 1992). Sobald die ersten 10 Epochen rücktransformiert sind, wird der erste Test durchgeführt, wobei die Reizantworten des rechten und des linken Ohres getrennt getestet werden. Simultan zum Testen läuft die Datenerfassung weiterer Epochen. Jede erfaßte Epoche wird mittels der FFT transformiert, rauschgefiltert und in den Zeitbereich rücktransformiert. Sobald zu den ersten 10 Epochen weitere 5 hinzu gekommen sind, wird erneut getestet. Dieser sequentielle Testablauf wird weitergeführt, bis beide ASSR (rechts und links) nachgewiesen worden sind oder, wenn nur eine ASSR oder keine ASSR nachweisbar ist, bis eine maximale Anzahl von 120 Epochen aufgelaufen ist. Für das Beispiel wird angenommen, daß die ASSR des rechten Ohres bereits mit 25 Epochen (25 Sekunden), die ASSR des linken Ohres nach 30 Epochen (30 Sekunden) nachgewiesen wird. Nach 25 Sekunden signalisiert das Screeninggerät für das rechte Ohr ein "Pass" und nach weiteren 5 Sekunden auch für das linke Ohr ein "Pass". Das simultane Hörscreening beider Ohren ist folglich nach 30 Sekunden beendet. Bei einer behandlungsbedürftigen Hörminderung eines oder beider Ohren beträgt die Testdauer bis zum Signal "Fail" 120 Sekunden, wobei bei einseitiger Schwerhörigkeit zuvor für das gesunde Ohr nach entsprechender kürzerer Testdauer ein "Pass" signalisiert wird. Die Untersuchung läuft jedoch weiter, bis das Ergebnis für beide Ohren vorliegt.

## Patentansprüche

1. Verfahren zum zum objektiven Nachweis von Auditory Steady-State Responses (ASSR) im Zeitbereich, wobei die einzelnen Epochen in den Frequenzbereich transformiert werden.

2. Verfahren nach anspruch 1, wobei im Frequenzbereich die Antwort vom Rauschen des Spontan-EEG befreit wird.

3. Verfahren nach anspruch 2, wobei die Amplituden der Spektrallinien, die nur Rauschen enthalten, auf Null gesetzt werden.

4. Verfahren nach anspruch 1, wobei die Spektrallinien der Harmonischen unbeeinflußt bleiben.

5. Verfahren nach anspruch 1-4, wobei das veränderte Spektrum wird in den Zeitbereich rücktransformiert.

6. Verfahren nach anspruch 1, wobei alle Spektrallinien, die nur Rauschen enthalten, weggelassen werden wobei, das Spektrum nur noch aus hauptsächlich den Harmonischen der Antwort besteht.

7. Verfahren nach anspruch 6, wobei das so veränderte Spektrum wird in den Zeitbereich rücktransformiert.

8. Verfahren nach anspruch 1-7, wobei die nach Rücktransformation in den Zeitbereich erhaltene Zeitfunktion der Antwort ein im Zeitbereich arbeitender statistischer Test angewendet wird.
